# EUROPEAN PATENT APPLICATION

(11) **EP 1 685 790 A1**
(43) Date of publication of application: **02.08.2006**
(21) Application number: 04818481.6
(22) Date of filing: 10.11.2004
(51) Int. Cl.: A61B 1/04

(54) **ENDOSCOPE DEVICE AND IMAGING METHOD USING THE SAME**

(30) Priority: 14.11.2003 JP 2003385205
(71) Applicant: Apricot Co., Ltd., Tokyo 164-0012 (JP)
(72) Inventor: Osaka, Shoji, Shinagawa-ku Tokyo 141-0031 (JP)
(74) Representative: Epping - Hermann - Fischer
(86) International application number: PCT/JP2004/016665
(87) International publication number: WO 2005/046462

(57) **Abstract**

Three objective lenses (11) having optical axes L oriented to different directions are mounted on a head portion (10) of an endoscope device. The objective lens (11) has a predetermined view angle θ centered by the optical axes L. Each objective lens (11) is disposed so as to have the peripheral portions of its viewing field overlap with the peripheral portions of the viewing fields of adjoining other objective lenses (11). Hence, the three objective lenses (11) causes the head portion (10) as a whole to have a view angle φ extending over a wider range than the view angle θ of the objective lens (11) with no discontinuation. Each optical image of a testing target part captured within this view angle φ is imaged by an imaging element.

## Description

### Technical Field

The present invention relates to an endoscope device for medical or industrial use, and an imaging method using the same.

### Background Art

Conventionally, a medical endoscope for capturing images inside a body cavity by inserting an elongate inserting unit into the body cavity (lumen) has been widely used.

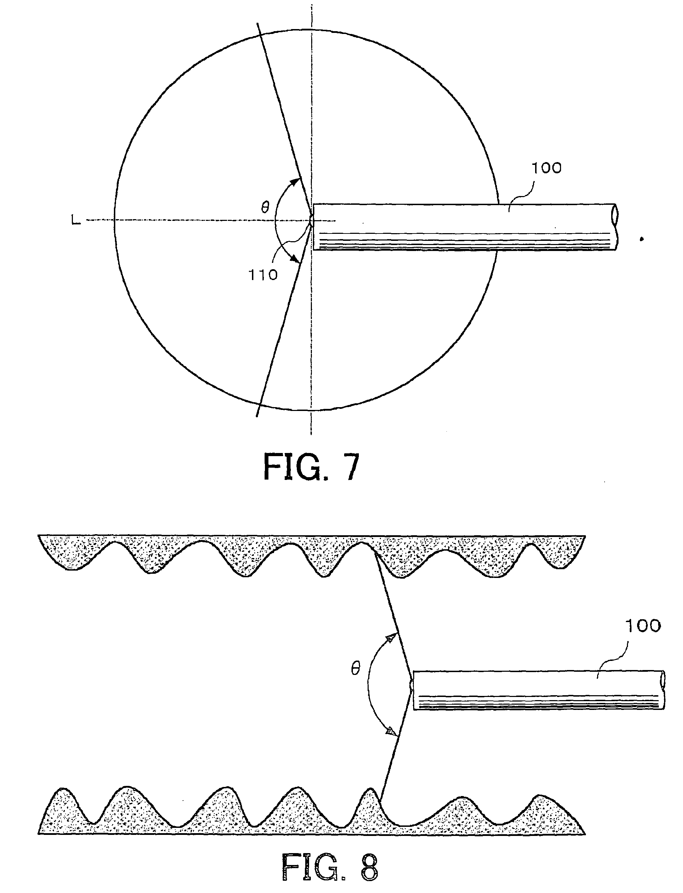

For example, as shown in FIG. 7, such an endoscope has an objective lens 110 at the head of its inserting unit 100. The objective lens 110 has an optical axis L defined in the longer axis direction, and catches the testing target part (observation part) ahead, in a predetermined view angle (for example, about 140 degrees) fanning out about the optical axis L. An optical image of the testing target part caught by the objective lens is formed on an imaging element such as a CCD (Charge Coupled Device), etc. via an unillustrated internally disposed lens mechanism (relay optical system, etc.).

That is, when the inserting unit 100 as shown in FIG. 7 is inserted into the body cavity, the endoscope can capture the image of the testing target part ahead of the inserting unit 100.

The inserting unit 100 has a curving portion free to curve formed of jointed curve pieces, and a limber flexible tubular portion adjoining the curving portion and having flexibility. With these, the inserting unit 100 is formed free to bend by a predetermined bending radius.

As disclosed in a Patent Literature 1, by an operation of an observer (for example, an operation onto an unillustrated operation unit at hand), the inserting unit 100 can be bent to an arbitrary direction and the direction of its viewing field can be desirably changed.

Such an endoscope is applied not only for medical use but only for industrial use. For example, since an endoscope can capture images inside the ducts of plant equipment or inside machines, etc., it is used for nondestructive test, etc.

As described above, an endoscope is formed free to bend. Hence, in a case where it is inserted into a lumen (for example, esophagus and stomach) having a larger inner diameter than the bending radius, an image of the lumen can be captured within the view angle of the objective lens, with the inserting unit 100 bent.

However, in a case where the inserting unit 100 is inserted into a lumen having a smaller inner diameter than its bending radius, the inserting unit 100 is hard to bend. Therefore, as shown in FIG. 8, the endoscope can only capture images of parts ahead of the objective lens within a view angle θ. That is, in a case where the inner wall of the lumen has folds, etc. thereon, there has been a problem that some parts (for example, the depth and back of a fold, etc.) cannot be captured because the view angle is narrow.

Even in the case where the inserting unit 100 can be bent, it has been necessary for the observer to adequately bend the inserting unit 100 in order to capture images around the inserting unit 100 over a wider range than the view angle θ (for example, over the full range of 360 degrees). The observer has to appropriately operate the operation unit at hand (operation knob, etc.).

However, this operation not only is complicated, but also requires some skill.

Further, even a skilled observer cannot simultaneously capture images of parts separate from each other by a predetermined distance or larger, though he/she can capture images over a wide range. That is, in order to capture images of two parts forming an angle therebetween larger than the view angle of the objective lens 110, it is necessary to stagger the timings to capture images.

Thus, a conventional endoscope has a problem that it cannot allow two parts distanced by a predetermined angle or larger to be simultaneously captured so that their independent behaviors and influences of the behaviors of one part onto the behaviors of the other may be imaged. That is, the conventional endoscope cannot perform image capturing that satisfies the observer's demands.
Patent Literature 1: Unexamined Japanese Patent Application KOKAI Publication No. H5-15484

### Disclosure of Invention

### Problem to be Solved by the Invention

The present invention was made in view of the above-described circumstance, and an object of the present invention is to provide an endoscope device and an imaging method capable of appropriately performing image capturing over a wide range.

### Means for Solving the Problem

To achieve the above object, an endoscope device according to a first aspect of the present invention is an endoscope device comprising an elongate inserting unit,
wherein the inserting unit comprises:
plurality of objective optical means, having a predetermined view angle, mounted so as to be oriented in different viewing field directions from one another;
transmission optical means for transmitting a light flux entering each of the objective optical means; and
   - imaging means for imaging respective optical images formed when the light flux transmitted by the transmission optical means is converged.

According to this structure, the plurality of objective optical means each have a predetermined view angle θ (for example, about 140 degrees), and are mounted so as to be oriented in different viewing field directions. The transmission optical means is formed of, for example, a prism, a relay lens, etc., and transmits a light flux entering each objective optical means. The imaging means is formed of, for example, a CCD, etc., and images each optical image formed when the light flux transmitted by the transmission optical means is converged.

As a result, image capturing over a wide range can be performed.

The objective optical means may be disposed so as to have peripheral portions of their respective viewing fields overlap peripheral portions of other viewing fields, and
the imaging means may image an optical image of a light flux captured within a view angle φ (for example, about 240 degrees) extending, with no discontinuation, over a wider range than the view angle of each objective optical means.

To achieve the above object, an endoscope device according to a second aspect of the present invention is an endoscope device comprising an elongate inserting unit,
wherein the inserting unit comprises:
three or more objective lenses, having a predetermined view angle, mounted so as to be oriented in different viewing field directions from one another;
a transmission optical system for transmitting light fluxes entering the respective objective lenses; and
an imaging element for imaging respective optical images formed when the light fluxes transmitted by the transmission optical system are converged on three or more regions.

According to this structure, the three or more objective lenses each have a predetermined view angle θ (for example, about 140 degrees), and are mounted to be oriented in different viewing field directions. The transmission optical system is formed of, for example, a prism, a relay lens, etc., and transmits light fluxes entering the respective objective lenses. The imaging element is formed of, for example, a CCD, and images respective optical images formed when the light fluxes transmitted by the transmission optical system are converged on three or more regions.

As a result, image capturing over a wide range can be appropriately performed.

The objective lenses may be disposed so as to have peripheral portions of their respective viewing fields overlap peripheral portions of other viewing fields, and
the imaging element may image, on three regions, optical images of light fluxes captured within a view angle φ (for example, about 240 degrees) extending, with no discontinuation, over a wider range than the view angle of each objective lens.

To achieve the above object, an imaging method according to a third aspect of the present invention is an imaging method by an endoscope device comprising an elongate inserting unit,
wherein the inserting unit comprises a plurality of objective optical systems having a predetermined view angle and mounted so as to be oriented in different viewing field directions from one another, a transmission optical system for transmitting a light flux entering each objective optical system, and an imaging element for imaging each optical image formed when the light flux transmitted by the transmission optical system is converged;
wherein the imaging method comprises:
capturing testing target parts, by the respective objective optical systems, within their respective viewing fields which partially overlap with viewing fields of adjoining other objective optical systems; and
simultaneously imaging, by the imaging element, optical images of the testing target parts captured within a view angle extending, with no discontinuation, over a wider area than the view angle of each objective optical system.
According to this method, each objective optical system captures a testing target part within its own viewing field which partially overlaps with the viewing fields of adjoining other objective optical systems, and the imaging element simultaneously images respective optical images formed when light fluxes within the range of a view angle φ extending, with no discontinuation, over a wider range than the view angle θ of each objective optical system are converged.
As a result, image capturing over a wide range can be appropriately performed.

### Effects of the Invention

According to the present invention, it is possible to provide an endoscope device and an imaging method capable of appropriately performing image capturing over a wide range.

### Brief Description of Drawings

[FIG. 1] It is a perspective diagram showing one example of an endoscope device according to an embodiment of the present invention.
[FIGS. 2] (a) is a front elevation of a head portion of the endoscope device of FIG. 1. (b) is a side elevation of the head portion of the endoscope device of FIG. 1. (c) is a perspective diagram of the head portion of the endoscope device of FIG. 1.
[FIGS. 3] (a) is an exemplary diagram for explaining a view angle θ of an objective lens. (b) is an exemplary diagram for explaining a view angle φ of the entire head portion.
[FIG. 4] It is a partial cross-sectional diagram for explaining an internal structure of the head portion.
[FIG. 5] It is an exemplary diagram for explaining image capturing of a lumen by the endoscope device.
[FIG. 6] It is a partial cross-sectional diagram for explaining an internal structure of the head portion according to another embodiment of the present invention.
[FIG. 7] It is an exemplary diagram for explaining a head portion of a conventional endoscope.
[FIG. 8] It is an exemplary diagram for explaining image capturing by the conventional endoscope.

### Explanation of Reference Numerals

- 1: inserting unit
- 2: operation unit
- 10: head portion
- 11: objective lens
- 12: prism
- 13: prism
- 14: relay lens
- 15: imaging element
- 20: curving portion
- 30: flexible tubular portion
- 40: operation knob
- 50: cable

### Best Mode for Carrying Out the Invention

An endoscope device according to an embodiment of the present invention will be explained below with reference to the drawings.

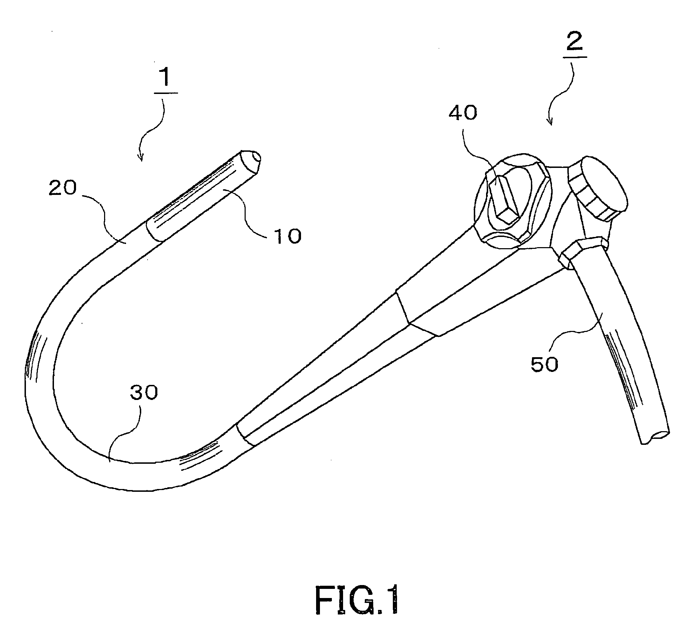

FIG. 1 is a perspective diagram showing the appearance of an endoscope device to be applied to the embodiment of the present invention. As shown, the endoscope device comprises an inserting unit 1, which is elongate and flexible, and an operation unit 2.

The inserting unit 1 comprises a head portion 10 in which a plurality of optical systems are disposed, a curving portion 20 free to curve formed of jointed curve pieces, and a limber flexible tubular portion 30 adjoining the curving portion 20 and having flexibility.

The operation unit 2 comprises an operation knob 40 and a cable 50.

The operation knob 40 is connected to the curving portion 20 by a known drive mechanism, and can curve the curving portion 20 to an arbitrary direction in response to a rotation (turn) of the operation knob 40.

The cable 50 is detachably connected to an unillustrated image processing apparatus, light source device, etc. The cable 50 supplies the image processing apparatus with video information originating from an optical image captured over the full circumference by the head portion 10 (on which an imaging element to be described later is disposed).

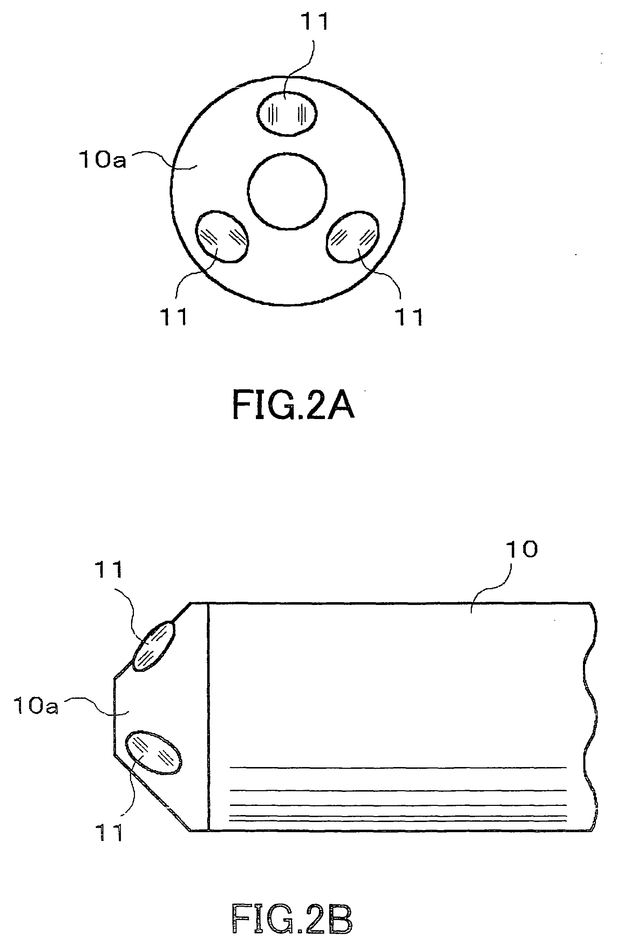

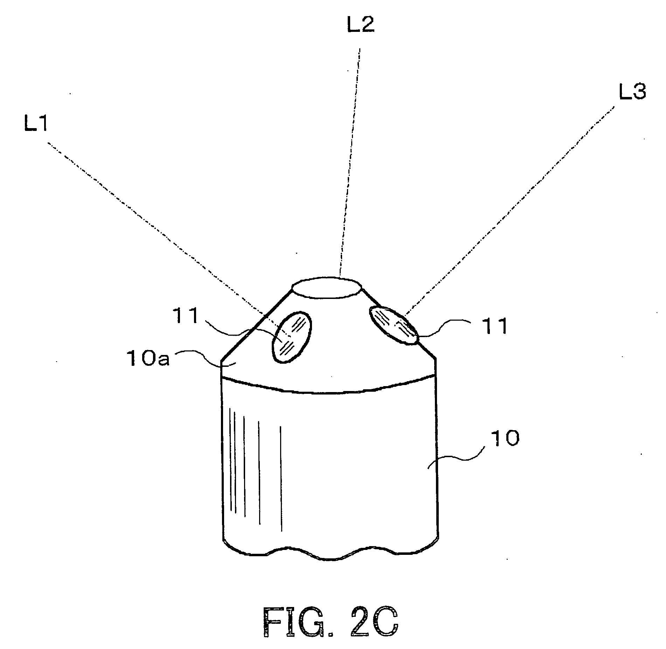

Next, the head portion 10 will be explained with reference to FIGS. 2, etc. FIG. 2A is a front elevation of the head portion 10, FIG. 2B is a side elevation of the head portion 10, and FIG. 2C is a perspective view of the head portion 10.

As shown in FIG. 2A to FIG. 2C, the head portion 10 has its head 10a formed generally like a truncated cone, and has three objective lenses 11 disposed equidistantly on the conic slope. The respective objective lenses are embedded in the slope of the head 10a and so disposed as to have their optical axes L1 to L3 in different directions from one another.

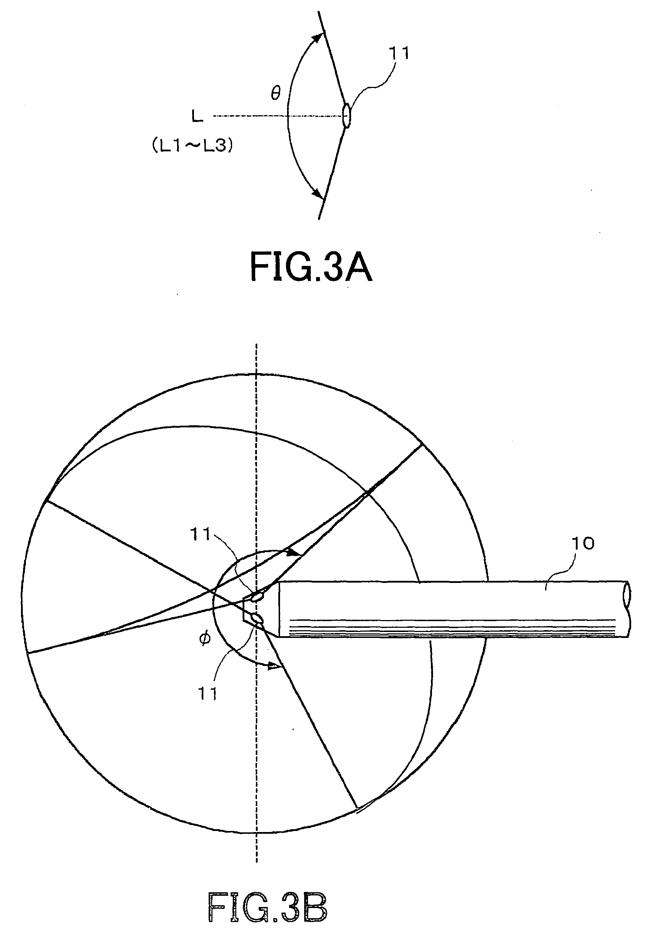

As shown in FIG. 3A, the objective lens 11 has a predetermined view angle θ (for example, about 140 degrees) centered by the optical axis L (L1 to L3).

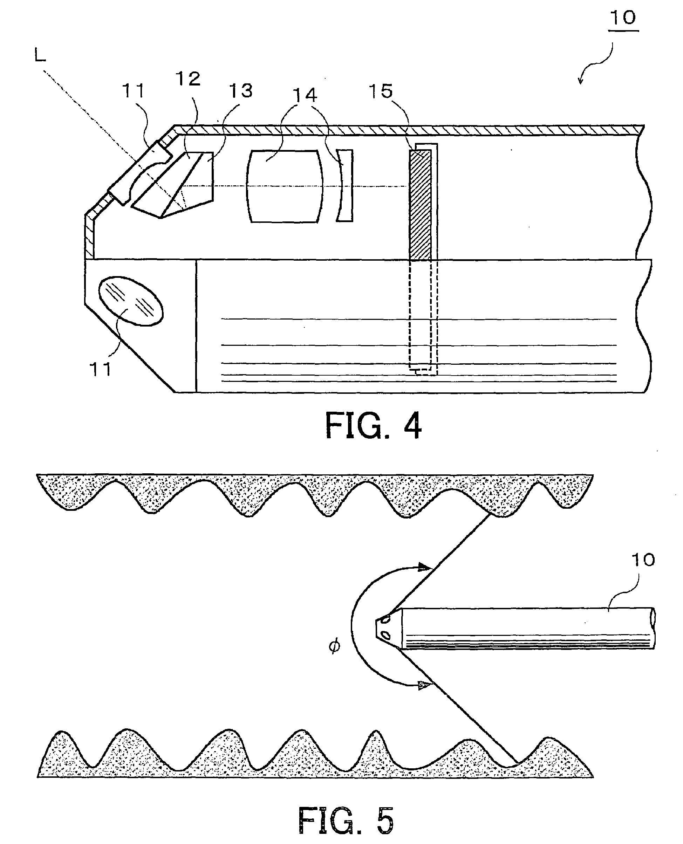

Here, the viewing field of each objective lens 11 is defined such that the peripheral portions thereof overlap with the peripheral portions of the other viewing fields, as shown in FIG. 3B. With the viewing fields of the three objective lenses combined, the head portion 10 has a view angle φ (an angle over 180 degrees, for example, about 240 degrees) that extends over a wide range with no discontinuation as a whole. [0033] The internal structure of the head portion 10 will be explained with reference to FIG. 4. FIG. 4 is a partial cross-sectional view of the head portion 10. Though FIG. 4 shows the cross section of one objective lens 11 and an optical system disposed with respect to this, the remaining two objective lenses and optical systems disposed with respect to these have also the same cross-sectional structure.

As shown, the objective lens 11, prisms 12 and 13, a relay lens 14, and an imaging element 15 are disposed in the head portion 10.

The objective lens 11 catches a testing target part (observation part) ahead, which is irradiated with illumination by an unillustrated illumination probe, within a predetermined view angle (for example, about 140 degrees) centered by the optical axis L, as described above. Then, each objective lens 11 supplies the light flux of the incoming reflection light from the testing target part to the prisms 12 and 13.

The prisms 12 and 13 refract the light flux entering the objective lens 11 at their respective angles, in order to converge the light, via the relay lens 14 behind, on the imaging element 15.

The relay lens 14 comprises a plurality of lenses and forms an optical image of the testing target part on the imaging surface of the imaging element 15 by letting in the light flux refracted by the prisms 12 and 13.

The imaging element 15 comprises a CCD (Charge Coupled Device), etc. having a latticed color filter disposed on the front face thereof, and opto-electrically converts the optical image supplied via the relay lens 14 and formed on the imaging surface into an electric signal.

The imaging element 15 has its imaging surface divided into three regions, and the light fluxes entering different objective lenses 11 (objective optical systems) are converged on these regions respectively.

The imaging element 15 supplies the video signal of each optical image opto-electrically converted into the electric signal to the image processing apparatus connected to the cable 50 of the operation unit 2 through a unillustrated signal line.

The operation of the endoscope device according to the embodiment of the present invention will be explained below.

Here, a case will be explained, where the inserting unit 1 (head portion 10) of the endoscope device is inserted into a relatively narrow lumen, as shown in FIG. 5.

The head portion 10 inserted into a lumen is adequately turned in response to an operation of the operation knob 40. As a result, the head portion 10 catches the testing target part (observation part) extending over a wider range than the view angle θ of the objective lens, by the three objective lenses 11 within the view angle φ (for example, 240 degrees).

As described above, with reference to FIG. 4, the light fluxes entering the respective objective lenses 11 are refracted by the prisms 12 and 13 and converged on the three regions on the imaging surface of the imaging element 15 via the relay lens 14.

That is, the light fluxes representing the optical images of the testing target parts entering the different object lenses 11 are converged on the three regions of the imaging surface of the imaging element 15 respectively.

The imaging element 15 converts the converged optical images into electric signals, and supplies video signals of the respective converted optical images to the image processing apparatus via the unillustrated signal line and the cable 50.

Then, when the image processing apparatus acquires the video signals, it applies a predetermined image process thereto and simultaneously displays the testing target parts caught by the respective objective lenses 11 by the plurality of videos corresponding to the respective parts. That is, the image processing apparatus displays the videos of the testing target parts caught by the three objective lenses 11 respectively in real time and simultaneously.

As described, even two testing target parts, which are distanced by larger than the view angle θ of one objective lens 11, can be image-captured simultaneously.

Accordingly, even if the inner walls of the lumen are folded as shown in FIG. 5, it is possible to capture even the depths and backs of the folds without bending the head portion 10.

Further, it becomes possible to simultaneously capture the independent behaviors of the two parts distanced by larger than the view angle θ and influences given by the behaviors of one part onto the behaviors of the other, enabling an image capturing that satisfies demands of the observer.

In the above-described embodiment, the case has been explained where the light fluxes entering the three optical systems are converged on the imaging surface made of divided regions of one imaging element. The number of the imaging element 15 is not limited to one.

For example, the same number of imaging elements 15 as the number of objective lenses may be disposed. In this case, it is possible to omit the prisms 12 and 13 by disposing each imaging element 15 in line with the direction of each objective lens (in line with orientation in which each is disposed).

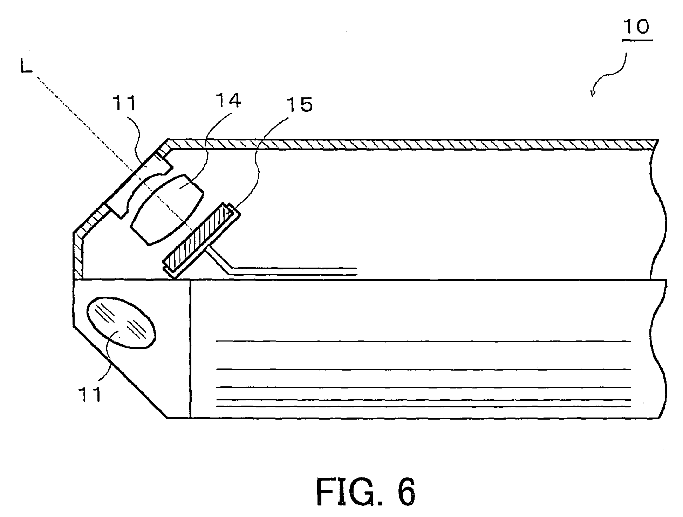

That is, the imaging device 15 is disposed in line with the direction of the objective lens 11 as shown in FIG. 6. That is, the imaging surface of the imaging device 15 is disposed so as to be orthogonal to the optical axis L of the objective lens 11. FIG. 6 shows only the cross section of one objective lens 11, and the relay lens 14 and imaging element 15 disposed with respect thereto. The other objective lenses also have corresponding relay lenses and imaging elements disposed coaxially.

In case of such disposition, the light flux entering the objective lens 11 goes straightforward and is converged on the imaging element 15 via the relay lens 14. That is, the prisms 12 and 13 can be omitted because there is no need of refracting the light flux.

In the above-described embodiment, a case has been explained where the images of the three testing target parts captured by the endoscope device are simultaneously displayed by the image processing apparatus. However, image selection means may be disposed between the imaging element 15 and the image processing apparatus, so that the video signal of an optical image selected from the optical images of the plurality of testing target parts imaged by the imaging element 15 may be output to the image processing apparatus.

Further, the image processing device may perform some image process based on the video signals of the respective optical images obtained from the imaging element 15 and generate a three-dimensional panoramic image.

That is, since the peripheries of the viewing field of each objective lens 11 overlap with the peripheries of the other adjoining viewing fields as shown in FIG. 3B, the head portion 10 has got a view angle φ (for example, about 240 degrees) extending over a wide range with no discontinuation. This enables the image processing apparatus to generate a three-dimensional panoramic image on which the images of the testing target parts dispersed over a wide range are developed, based on the respective obtained optical images. Since this three-dimensional panoramic image has a relatively small data size, it is an easy option to use this image for electronic medical chart. Further, by reproducing a stored three-dimensional panoramic image, a detailed endoscopic video full of live feeling like, for example, a stereovision generated by stereoscopically converging respective optical images, will be reproduced.

The generation of a three-dimensional panoramic image may be realized not by an image processing apparatus but by a circuit board possessed by the imaging element 15.

In the above-described embodiment, a case has been explained where three optical systems (three objective lenses 11, and prisms 12 and 13 and relay lenses 14 corresponding to the respective objective lenses 11) are disposed at the head portion 10. However, the number of these optical systems needs only to be equal to or larger than two, and is not limited to three.

In a case where objective lenses having a wide view angle are used, even if the number of such optical systems is, for example, two, the viewing fields of the respective objective lenses overlap each other partially, making it possible to secure a viewing field having a wide angle.

On the other hand, in a case where objective lenses having a narrow view angle are used, the number of such optical systems may be increased to, for example, four or larger and disposed at the head portion 10, in order to secure overlapping portions of the viewing fields adjoining each other.

In the above-described embodiment, a case has been explained where the imaging element 15 is disposed inside the head portion 10. However, the imaging element may be disposed in the operation unit 2. A light flux entering the objective lens 11 is converted into an optical signal and transmitted through, for example, a fiber optic cable, and demodulated by an unillustrated demodulation unit. The demodulated optical signal is supplied to the imaging element in the operation unit 2 and converged on the imaging surface of the imaging element as the optical image of the testing target part.

In this case, the head portion 10 can be downsized.

In the above-described embodiment, a case has been explained where the least components necessary for capturing images are disposed in the head portion 10, in order to facilitate understanding. However, for example, a component for irradiating an illumination light, etc. may be disposed in the head portion 10. In this case, an illumination lens for irradiating the testing target part with an illumination light is disposed at the head 10 or therearound. An illumination light supplied from an unillustrated light source device connected to the cable 50 is transmitted to the illumination lens through a fiber optic, etc. A plurality of illumination lenses may be disposed correspondingly to the objective lenses 11.

In this case, an illumination probe, etc. becomes unnecessary at the time of capturing images.

A person skilled in the art could add various modifications onto the above-described embodiment, without departing from the sprit and scope of the present invention. The above-described embodiment is intended for illustration, not to limit the scope of the present invention. Accordingly, the scope of the present invention should be determined not by referring to the above description, but in accordance with the entire scope of equivalents to which the claims shown below are entitled.

The present invention based on Japanese Patent Application No. 2003-385205 filed on November 14, 2003, and including specification, claims, drawings and summary. The disclosure of the above Japanese Patent Application is incorporated herein by reference in its entirety.

### Industrial Applicability

As explained above, according to the present invention, it is possible to provide an endoscope device, etc. capable of appropriately performing image capturing over a wide range.

## Claims

1. An endoscope device comprising an elongate inserting unit (10),
wherein said inserting unit (10) comprises:
plurality of objective optical means (11), having a predetermined view angle, mounted so as to be oriented in different viewing field directions from one another;
transmission optical means (12, 13, 14) for transmitting a light flux entering each of said objective optical means (11); and
imaging means (15) for imaging respective optical images formed when the light flux transmitted by said transmission optical means (12, 13, 14) is converged.

2. The endoscope device according to claim 1,
wherein:
said objective optical means (11) are disposed so as to have peripheral portions of their respective viewing fields overlap peripheral portions of viewing fields of adjoining other objective optical means (11); and
said imaging means (15) images an optical image of a light flux captured within a view angle extending, with no discontinuation, over a wider range than the view angle of each objective optical means (11).

3. An endoscope device comprising an elongate inserting unit (10),
wherein said inserting unit (10) comprises:
three or more objective lenses (11), having a predetermined view angle, mounted so as to be oriented in different viewing field directions from one another;
a transmission optical system (12, 13, 14) for transmitting light fluxes entering said respective objective lenses (11); and
an imaging element (15) for imaging respective optical images formed when the light fluxes transmitted by said transmission optical system (12, 13, 14) are converged on three or more regions.

4. The endoscope device according to claim 3,
wherein:
said objective lenses (11) are disposed so as to have peripheral portions of their respective viewing fields overlap peripheral portions of viewing fields of adjoining other objective lenses (11); and
said imaging element (15) images, on at least three regions, optical images of light fluxes captured within a view angle extending, with no discontinuation, over a wider range than the view angle of each objective lens (11).

5. An endoscope device comprising an elongate inserting unit (10) and an operation unit (2) connected to said inserting unit (10), wherein said endoscope device comprises:
plurality of objective optical means (11), disposed at said inserting unit (10), having a predetermined view angle, and mounted so as to be oriented in different viewing field directions from one another;
transmission optical means (12, 13, 14), disposed at said inserting unit (10), for transmitting light fluxes entering said respective objective optical means (11);
light carrying means, disposed at said inserting unit (10), for carrying the light fluxes transmitted by said transmission optical means (12, 13, 14) between said inserting unit (10) and said operation unit (2) toward imaging means; and
imaging means (15), disposed at said operation unit (2), for imaging respective optical images formed when the light fluxes transmitted by said transmission optical means (12, 13, 14) and carried by said light carrying means are converged.

6. An endoscope device comprising an elongate inserting unit (10),
wherein said inserting unit (10) comprises:
plurality of objective optical means (11), having a predetermined view angle, disposed so as to have different viewing field directions from one another;
transmission optical means (12, 13, 14), having different optical axes from one another, for independently transmitting light fluxes entering said respective objective optical means (11) along the optical axes; and
imaging means (15) for imaging respective optical images formed when the light fluxes transmitted by said transmission optical means (12, 13, 14) are converged.

7. The endoscope device according to claim 6,
wherein said endoscope device further comprises image selection means for selecting a part of the optical images imaged by said imaging means (15).

8. The endoscope device according to claim 6,
wherein said endoscope device further comprises three-dimensional image generation means for generating a three-dimensional panoramic image by combining the respective optical images converged by said imaging means (15).

9. An endoscope device comprising an elongate inserting unit (10),
wherein said inserting unit (10) comprises:
illumination means for irradiating a testing target part with a ray of light supplied from a predetermined light source device;
plurality of objective optical means (11), mounted so as to be oriented in different viewing field directions from one another, for receiving a reflection light of the ray of light irradiated by said illumination means, within a predetermined view angle;
transmission optical means (12, 13, 14) for transmitting a light flux entering each of said objective optical means (11); and
imaging means (15) for imaging each optical image formed when the light flux transmitted by said transmission optical means (12, 13, 14) is converged.

10. An imaging method by an endoscope device comprising an elongate inserting unit (10),
wherein said inserting unit (10) comprises a plurality of objective optical systems (11) having a predetermined view angle and mounted so as to be oriented in different viewing field directions from one another, a transmission optical system (12, 13, 14) for transmitting a light flux entering each objective optical system (11), and an imaging element (15) for imaging each optical image formed when the light flux transmitted by said transmission optical system (12, 13, 14) is converged;
wherein said imaging method comprises:
capturing testing target parts, by said respective objective optical systems (11), within their respective viewing fields which partially overlap with viewing fields of adjoining other objective optical systems (11); and
simultaneously imaging, by said imaging element (15),optical images of the testing target parts captured within a view angle extending, with no discontinuation, over a wider area than the view angle of each objective optical system.

11. An imaging method by an endoscope device and an imaging device optically connected to said endoscope device,
wherein said imaging method comprises:
preparing an endoscope device, in which a plurality of objective optical systems (11) mounted so as to be oriented in different viewing field directions from one another for receiving, within a predetermined view angle, reflection lights from testing target parts irradiated with an illumination light, and a transmission optical system (12, 13, 14) for transmitting light fluxes entering said respective objective optical systems (11), are disposed;
capturing, by said respective objective optical systems (11), the testing target parts, within respective viewing fields which partially overlap with viewing fields of adjoining other objective optical system (11); and
receiving, by an imaging device (15), optical signals of optical images of the testing target parts formed when light fluxes transmitted by said transmission optical system (12, 13, 14) and carried from said endoscope device are converged, and demodulating the optical signals and imaging the optical images of the testing target parts.

12. An imaging method by an endoscope device comprising an elongate inserting unit (10) and an operation unit (2) connected to said inserting unit via a connection unit,
wherein:
objective optical systems (11) having a predetermined view angle and mounted so as to be oriented in different viewing field directions from one another, and a transmission optical system (12, 13, 14) for transmitting light fluxes entering said respective objective optical systems (11) are disposed at said inserting unit (10);
light carrying means for converting light fluxes transmitted by said transmission optical system (12, 13, 14) into optical signals and carrying them is disposed at said connection unit; and
demodulation means for demodulating the optical signals carried by said light carrying means, and an imaging element (15) for receiving the optical signals demodulated by said demodulation means and imaging optical images based on the optical signals are disposed at said operation unit (2),
wherein said imaging method comprises:
capturing, by said respective objective optical systems (11), testing target parts within respective viewing fields which partially overlap with viewing fields of adjoining other objective optical systems (11); and
imaging, by said imaging element (15), optical images of the testing target parts based on optical signals originating from demodulated light fluxes transmitted by said transmission optical system (12, 13, 14).
